(19)

Europäisches
Patentamt
European
Patent Office
Office européen
des brevets

(11) **EP 1 611 845 B1**

(12) **EUROPEAN PATENT SPECIFICATION**

(45) Date of publication and mention
of the grant of the patent:
**18.07.2012 Bulletin 2012/29**

(51) Int Cl.:
***A61B 5/0424*** *(2006.01)*

(21) Application number: **05105795.8**

(22) Date of filing: **29.06.2005**

(54) **An electrode connectivity determination system**

System zur Bestimmung einer Elektrodenverbindung

Système pour la détermination de l'existence d'une connexion d'une électrode

(84) Designated Contracting States:
**DE FR GB**

(30) Priority: **29.06.2004 US 583812**

(43) Date of publication of application:
**04.01.2006 Bulletin 2006/01**

(73) Proprietor: **Draeger Medical Systems, Inc.
Danvers, MA 01923 (US)**

(72) Inventor: **Prydekker, Randolph
Wilmot, NH 03827, (US)**

(74) Representative: **Kietzmann, Lutz
Maiwald Patentanwalts GmbH
Benrather Strasse 15
40213 Düsseldorf (DE)**

(56) References cited:
**EP-A- 1 275 342    US-A- 4 459 993
US-A- 4 577 639    US-A- 4 993 423
US-A- 5 704 351**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a system for determining the connectivity of electrodes, and in particular for determining the connectivity of electrodes to a patient in a medical monitoring and/or treatment device.

BACKGROUND OF THE INVENTION

Patient monitoring and/or treatment systems often use patient attachable electrodes. As one example, ECG systems are well known and provide information about the physiological status of a patient's heart to a physician. So-called conventional 12 lead ECG systems exist (both wired and wireless) which provide twelve waveforms, called ECG leads or lead signals, to a physician. To provide such a 12 lead ECG, ten patient attachable electrodes are attached to predefined locations on a patient's body, and the signals from these patient attachable electrodes are processed to provide the twelve ECG lead signals. The respective ECG lead signals are based on differential signals derived from two or more of the signals from patient attachable electrodes, in a known manner. These ten patient attachable electrodes include four patient attachable electrodes which provide signals that are processed to generate six limb ECG lead signals, and six patient attachable electrodes which provide signals that are processed to provide six precordial or chest ECG lead signals. It is also possible that a subset or reduced number of patient attachable electrodes may be used, compared to a conventional 12 ECG lead signal, for example, if surgery or injury prevents attachment of a patient attachable electrode at a predefined location. For example, a six ECG lead system may be generated by attaching six electrodes. It is also possible that a superset or additional number of patient attachable electrodes may be used, compared to a conventional 12 ECG lead system to provide additional data to a physician about a patient cardiac function.

A patient attachable electrode signal may become at least potentially degraded if the electric al contact between the patient attachable electrode and a patient body is impaired. Such an impaired electrical contact may occur if (a) the patient attachable electrode is detached from said patient body or (b) the electrical resistivity between the patient attachable electrode and the patient body is significantly increased because of degraded electrical contact. When the signal from a patient attachable electrode becomes degraded, one or more ECG lead signals based on the electrical signal from that patient attachable electrode may be inaccurate.

US 4, 459,993 discloses a method and apparatus for monitoring persons heartbeat activity and producing an estimate of heartbreak rate. First and second electrodes are positioned in contact with the patient. A differential amplifier detects and amplifies EGK signals received from the leads and provides the signal to a heartbeat detector and continuity detector. The amplified signal from the differential amplifier is provided to a bandpass detector and is rectified to produce dc level signal. The dc level signal is than compared to a threshold in threshold detector. If the dc level signal exceeds the threshold an inhabit signal is generated.

Existing patient monitoring and/or treatment devices such as ECG measurement systems, incorporate some form of decision making related to the suitability of ECG lead signals for algorithm processing and display. One known method for detecting this open circuit condition is by using a resistive divider and comparator scheme. In this scheme typically a resistive divider converts the series resistance of skin, patient attachable electrode, wire and processing circuitry to a measurable signal. More specifically, the measurable signal is a low frequency or DC signal. A comparator determines when this measurable signal exceeds a predetermined threshold value. When the threshold value is exceeded the display and processing of an affected ECG lead or leads based on the patient attachable electrode is inhibited. As used herein, a comparator is a circuit which compares a first signal to a second and generates a signal representing the result of the comparison. Typically the signal is a bistate signal having a first state when the first signal is greater than or equal to the second signal and a second state when the first signal is less than the second signal.

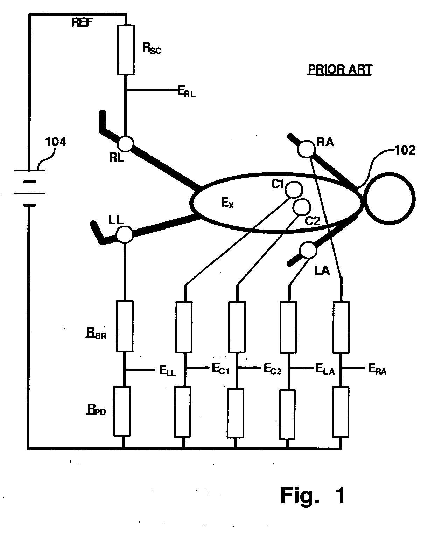

Fig. 1 is a diagram illustrating schematically the arrangement of patient attachable electrodes on a patient 102 and a portion of electrical circuitry associated with the electrodes. In Fig. 1, six patient attachable electrodes, right leg RL, left leg LL, right arm RA, left arm LA, first chest C1 and second chest C2, are illustrated by circles. These are representative only and illustrated to simplify the figure. One skilled in the art understands that other patient attachable electrodes (not shown) may be attached to the patient 102 at other predefined locations.

An electric voltage source 104, illustrated in Fig. 1 as a battery, has a first terminal coupled to a reference patient attachable electrode RL and patient attachable electrode terminal $E_{RL}$ via a first resistance $R_{SC}$, in a common branch. The second terminal of the battery 104 is coupled in common to the other patient attachable electrodes, LL, LA, RA, C1, C2, via respective series connections of pull-down resistors $R_{PD}$, patient attachable electrode terminals, $E_{LL}$, $E_{LA}$, $E_{RA}$, $E_{C1}$, $E_{C2}$, and branch resistors $R_{BR}$ in corresponding non-common branches. The electrode signal terminals $E_{RL}$, $E_{LL}$, $E_{LA}$, $E_{RA}$, $E_{C1}$, $E_{C2}$ are coupled to ECG processing and display circuitry (not shown). In the description below, $E_{LL}$, $E_{LA}$, $E_{RA}$, $E_{C1}$, $E_{C2}$, are used to designate the patient attachable electrode terminals, and the signals produced at those terminals. One skilled in the art understands from the context whether the signal or terminal is referred to.

In operation, the voltage source 104 produces a voltage REF at the first terminal with respect to the voltage at the second terminal. The reference patient attachable electrode RL is generally maintained at a constant potential, and establishes a reference potential on the patient 102 at the location of the RL patient attachable electrode. The reference patient attachable electrode terminal $E_{RL}$ produces this reference voltage and is coupled to the processing circuitry (not shown) to provide reference and monitoring.

The ECG signal voltages at the non-common patient attachable electrode terminals, $E_{LL}$, $E_{LA}$, $E_{RA}$, $E_{C1}$, $E_{C2}$, are measured with respect to the reference voltage REF at terminal $E_{RL}$ and are processed by the ECG processing circuitry (not shown) to produce differential ECG lead signals (I, II, V, V+, etc.) which are displayed for a physician. For example, the I lead signal is generated by subtracting the signal $E_{RA}$ from $E_{LA}$, the II lead signal is generated by subtracting the signal $E_{RA}$ from $E_{LL}$, and so forth. The patient attachable electrode signals, $E_{LL}$, $E_{LA}$, $E_{RA}$, $E_{C1}$, $E_{C2}$, are also processed to produce respective signals indicating that a patient attachable electrode signal is potentially degraded and that ECG lead signals derived from that patient attachable electrode are also potentially degraded.

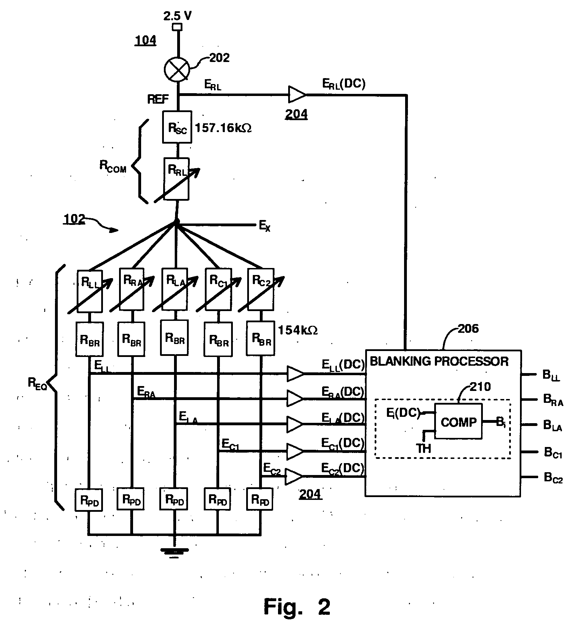

Fig. 2 is a more detailed electrical schematic diagram of an equivalent circuit of the electrode connectivity determination system. Elements in Fig. 2 which are the same as those in Fig. 1 are designated by the same reference number and are not described in detail below. The voltage source 104 is represented by a source of reference voltage, which in the illustrated embodiment is 2.5 volts, and a clamp 202. This generates a controlled reference voltage REF which is coupled to the reference electrode signal terminal $E_{RL}$, and to the RL patient attachable electrode through Rsc.

In Fig. 2, the resistivity of the respective patient attachable electrodes, RL, LL, RA, LA, C1, C2, are represented by variable resistances $R_{RL}$, $R_{LL}$, $R_{RA}$, $R_{LA}$, $R_{C1}$, and $R_{C2}$. Ideally, these resistances are relatively low or zero. In the case of a patient attachable electrode separating from a patient, these resistances become an open circuit. In the illustrated embodiment $R_{SC}$ is 157.16 kΩ, $R_{BR}$ is 154 kΩ, and $R_{PD}$ is a relatively high impedance.

In Fig. 2, the patient 102 (Fig. 1) is represented by connecting the patient attachable electrode resistance $R_{RL}$ to the respective patient attachable electrode resistances $R_{LL}$, $R_{RA}$, $R_{LA}$, $R_{C1}$, $R_{C2}$. There is a location within the patient at which the voltage $E_X$ exists. The combination of $R_{SC}$ in the common branch and the respective $R_{BR}$ and $R_{PD}$ resistances in the non-common branches form corresponding resistor dividers to which the non-common branch patient attachable electrode terminals, $E_{LL}$, $E_{LA}$, $E_{RA}$, $E_{C1}$, $E_{C2}$, are coupled. The patient attachable electrode terminals, $E_{RL}$, $E_{LL}$, $E_{LA}$, $E_{RA}$, $E_{C1}$, $Ec2$, are also coupled to a plurality of buffer amplifiers and low pass filters 204 which generate low frequency or DC signals $E_{RL}(DC)$, $E_{LL}(DC)$, $E_{RA}(DC)$, $E_{LA}(DC)$, $E_{C1}(DC)$, $E_{C2}(DC)$, representing the low frequency or DC component of the patient attachable electrode signals, $E_{RL}$, $E_{LL}$, $E_{LA}$, $E_{RA}$, $E_{C1}$, $E_{C2}$. The low frequency or DC patient attachable electrode signals, $E_{RL}(DC)$, $E_{LL}(DC)$, $E_{RA}(DC)$, $E_{LA}(DC)$, $E_{C1}(DC)$, $E_{C2}(DC)$, are coupled to a blanking processor 206.

If a patient attachable electrode, RL, LL, RA, LA, C1, C2, detaches from a patient, an open circuit exists in the voltage dividers. For example, if the reference patient attachable electrode RL detaches from the patient, then the voltage dividers have an open circuit. In this case, the DC level of the reference patient attachable electrode terminal $E_{RL}$ remains at the reference voltage REF, and that of the non-common patient attachable electrode terminals are pulled down to ground. If a non-common patient attachable electrode, LL, LA, RA, C1, C2, detaches from the patient then the DC level of that patient attachable electrode signal, $E_{LL}(DC)$, $E_{LA}(DC)$, $E_{RA}(DC)$, $E_{C1}(DC)$, $E_{C2}(DC)$, is pulled down to ground. The DC levels of the other non-common branch patient attachable electrode signals, $E_{LL}(DC)$, $E_{LA}(DC)$, $E_{RA}(DC)$, $E_{C1}(DC)$, $E_{C2}(DC)$, change due to the open circuit caused by the detached patient attachable electrode, LL, LA, RA, C1, C2 but remain close to their typical DC levels.

The blanking processor 206 includes a plurality of comparators 210, having respective first input terminals responsive to corresponding low frequency or DC patient attachable electrode signals $E_i(DC)$, second input terminals coupled to a source of a threshold signal TH, and output terminals coupled to corresponding blanking output terminals $B_i$. If, as described above, a patient attachable electrode has detached, then one or more of the non-common patient attachable electrode terminals are pulled down to ground. The threshold TH is set to detect this condition. If a patient attachable electrode signal exceeds the threshold, then the corresponding blanking signal is set to a first state indicating that the patient attachable electrode signal is degraded. Otherwise, i.e. the patient attachable electrode signal is within the threshold, the corresponding blanking signal is set to a second state indicating that the patient attachable electrode signal is not degraded. ECG processing circuitry receives the respective blanking signals $B_{LL}$, $B_{PA}$, $B_{LA}$, $B_{C1}$, $B_{C2}$ and blanks ECG lead signals depending on degraded patient attachable electrode signals. The blanking processor 206 may also include circuitry to determine which ECG lead signals are affected by the respective patient attachable electrode signals, and may generate ECG lead blanking signals generated so that any ECG lead signal which depends on a degraded patient attachable electrode signal is blanked.

This scheme works properly for patient attachable electrodes which detach from a patient. However, there are other situations in which the resistivities of the patient attachable electrodes increase without becoming an open circuit. For example, patient attachable electrodes dry out over time, causing their resistivity to increase. Also, the skin of older patients is relatively dryer than younger patients. This increases the apparent resistivity of the patient attachable electrodes. Increases in resistivity of one or more patient attachable electrode change the low frequency or DC component of patient attachable electrode signals for those patient attachable electrodes.

The divider/comparator scheme, based on a single threshold criteria, as described above, does not accommodate the fact that the DC voltage for a given patient attachable electrode is a function of a variety of different patient attachable electrode resistances and patients. That is, different patient attachable electrodes may have different electrode resistances and different patients have different skin conductivity, which affect the operation of the divider/comparator scheme. As a consequence, the divider/comparator scheme may work adequately in those cases when a ECG lead falls off. But in other circumstances involving different combinations of patient attachable electrode and/or patient skin resistance, decisions determining whether ECG signal data is to be processed and displayed become less reliable. In general, using such a divider/comparator scheme, if a threshold is chosen to have too high a value, excessively noisy data may be displayed and processed, and if too low a threshold value is selected, good data is inhibited from being processed and displayed.

There are several situations which may adversely affect the operation of the single threshold divider/comparator scheme. For example, as patient attachable electrodes are used over a relatively long period of time, they dry out. Referring again to Fig. 2, in this situation, this concurrently increases the resistances, $R_{RL}$, $R_{LL}$, $R_{LA}$, $R_{RA}$, $R_{C1}$, $R_{C2}$, of the patient attachable electrodes, RL, LL, LA, RA, C1, C2. This changes the parameters of the respective voltage dividers and decreases the DC voltages for the non-common patient attachable electrodes. However, the differential amplifiers (not shown) generating the ECG lead signals are still operating within acceptable constraints. Similarly, the resistance $R_{RL}$ of the patient attachable electrode in the common branch, or the resistance, $R_{LL}$, $R_{LA}$, $R_{RA}$, $R_{C1}$, $R_{C2}$, of one of the non-common patient attachable electrodes may increase, due, for example, to impaired electrical contact with the patient and/or dry skin on a patient leading to similar operations as described above. The prior art system would blank the non-common patient attachable electrode signals , $F_{LL}$, $E_{LA}$, $E_{RA}$, $E_{C1}$, $E_{C2}$, even though the ECG lead signals derived from them are being generated accurately. A system according to invention principles addresses these limitations and associated problems.

BRIEF SUMMARY OF THE INVENTION

In accordance with principles of the present invention as defined in claims 1 and 15, an electrode connectivity determination system is coupled to a plurality of patient attachable electrodes. Such a system includes a comparator for performing a comparison of a low frequency differential signal derived from at least two of the patient attachable electrodes with a predetermined threshold signal and a decision processor for generating a signal indicating an electrode signal is at least potentially degraded in response to said first comparison.

BRIEF DESCRIPTION OF THE DRAWING

In the drawing:

Fig. 1 is a diagram partially in schematic form and partially in physical form illustrating the arrangement of patient attachable electrodes on a patient and a portion of electrical circuitry associated with the electrodes;

Fig. 2 is a schematic diagram of an equivalent circuit representing a portion of the electrode connectivity determination system illustrated in Fig. 1;

Fig. 3a, and Fig. 3b are block diagrams of a portion of the blanking processor illustrated in Fig. 2;

Fig. 4 is a block diagram of an arrangement of the system illustrated in Fig. 1, Fig. 2, Fig. 3a, and Fig. 3b in which a remote portion and a central monitor are linked via a communications channel; and

Fig. 5 is a flow chart illustrating the processing of patient attachable electrode signals to determine connectivity of electrodes on a patient.

DETAILED DESCRIPTION OF THE INVENTION

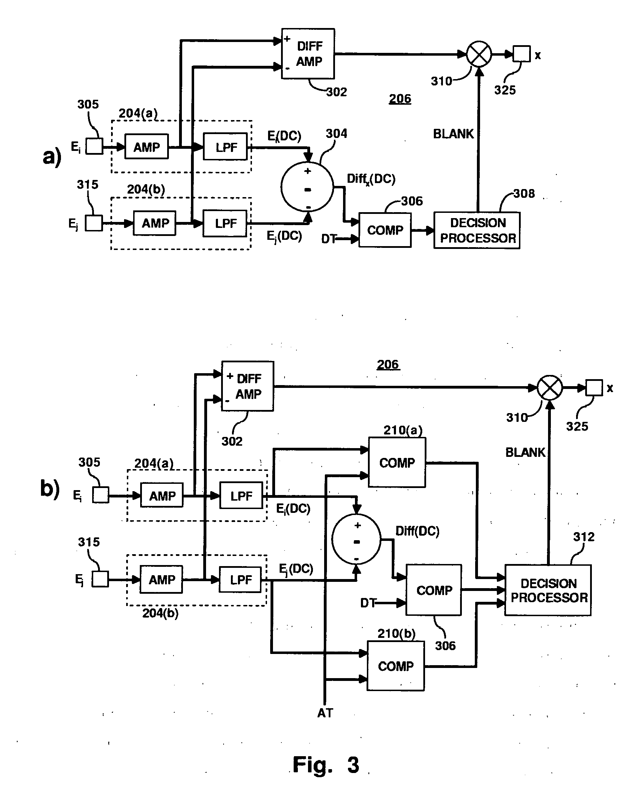

Fig. 3 is a block diagram of a portion of the blanking processor 206 illustrated in Fig. 2. In Fig. 3, those elements which are the same as those in Fig. 2 are designated by the same reference number and are not described in detail. In Fig. 3a, a first input terminal 305 and a second input terminal 315 are coupled to appropriate ones, $E_i$ and $E_j$, of the plurality, $E_{RL}$, $E_{LL}$, $E_{LA}$, $E_{RA}$, $E_{C1}$, $E_{C2}$, of patient attachable electrode terminals (Fig. 2) as described below. The first input terminal 305 is coupled to an input terminal of a first amplifier AMP. The first amplifier AMP is a unity gain buffer amplifier and is coupled to a non-inverting input terminal of a differential amplifier 302 and an input terminal of a first LPF. The combination of the first buffer amplifier AMP and first low pass filter LPF form the first buffer amplifier and LPF 204(a) (of Fig. 2). The second input terminal 315 is coupled to an input terminal of a second amplifier AMP. The second amplifier AMP is also a unity gain buffer amplifier and is coupled to an inverting input terminal of the differential amplifier 302 and an input terminal of a second LPF. The combination of the second amplifier AMP and second low pass filter LPF form the second buffer amplifier and LPF 204(b). An output terminal of the differential amplifier 302 is coupled to a signal input terminal of a blanking circuit 310. An output terminal of the blanking circuit 310 is coupled to an output terminal 325. The output terminal 325 generates one, x, of the respective ECG lead signals, i.e. I, II, V, V+, etc., and is coupled to ECG lead signal processing and display circuitry (not shown). Respective output terminals of the first and second buffer amplifiers and LPFs 204(a) and 204(b) are coupled to subtrahend and minuend input terminals of a subtractor 304. A difference output terminal of the subtractor 304 is coupled to a signal input terminal of a comparator 306. A source of a differential threshold signal DT is coupled to a comparison input terminal of the comparator 306. An output terminal of the comparator 306 is coupled to an input terminal of a decision processor 308. An output terminal of the decision processor 308 is coupled to a control input terminal of the blanking circuit 310.

In operation, the differential amplifier 302 generates a differential signal representing an ECG lead signal, from the patient attachable electrode signals supplied to it. More specifically, in the illustrated embodiment, the I lead signal is calculated by coupling the $E_{LA}$ patient attachable electrode terminal to the first input terminal 305 and the $E_{RA}$ patient attachable electrode terminal of the second input terminal 315, thus subtracting the RA patient attachable electrode signal from the LA patient attachable electrode signal. Similarly, the II lead signal is calculated by coupling the $E_{LL}$ patient attachable electrode terminal to the first input terminal 305 and coupling the $E_{RA}$ patient attachable electrode terminal to the second input terminal 315, thus subtracting the RA patient attachable electrode signal from the LL patient attachable electrode signal.

One skilled in the art understands that calculation of other ECG lead signals requires calculation of intermediate signals and forming a differential signal in response to that intermediate signal. For example, an intermediate signal W is generated to be the average of the limb patient attachable electrode signals, $E_{RA}$, $E_{LA}$, and $E_{LL}$. That is

$$W = \frac{E_{LL} + E_{RA} + E_{LA}}{3}.$$

The V lead signal is calculated by generating the W signal as described above, coupling the $E_{C1}$ patient attachable electrode terminal of the first input terminal 305 and W to the second input terminal, thus subtracting the W signals from the C1 patient attachable electrode signal. Similarly, the V+ lead is calculated by generating the W signal as described above, coupling the $E_{C2}$ patient attachable electrode terminal of the first input terminal 305 and W to the second input terminal, thus subtracting the W signal from the C2 patient attachable electrode signal. One skilled in the art understands how to design and implement a circuit to properly calculate the required intermediate signal W.

One skilled in the art also understands that the differential amplifier 302 generates an output signal accurately provided that it is operated within known constraints. One such constraint is that the input signals must remain within the limits of the power supplied to the amplifier. That is, the input signal may not exceed the voltage of the power or ground levels. Typical differential amplifiers 302 also include a baseline recovery or autoblock circuit. This circuit removes a DC offset in the output signal from the differential amplifier. However, the baseline recovery or autoblock circuit can saturate and cease to operate accurately. To prevent saturation of the autoblock circuit, the difference between the DC levels of the respective input signals may not exceed a predetermined level. Other constraints exist and are understood by those skilled in the art.

The respective buffer amplifiers and LPFs 204(a) and 204(b) buffer and low pass filter the patient attachable electrode signals $E_i$ and $E_j$. They produce low frequency or DC signals $E_i(DC)$ and $E_j(DC)$. These low frequency or DC signals are subtracted in the subtractor 304 to produce a low frequency or DC differential signal $Diff_x(DC)$. The subtraction corresponds to the processing performed by the differential amplifier 302. Thus, the signal $Diff_x(DC)$ is the low frequency or DC component of the ECG lead signal x produced by the differential amplifier 302.

The differential threshold signal DT is set to a level which approximates the level at which the autoblock function activates in the differential amplifier 302. If the output signal from the comparator 306 indicates that the differential threshold was exceeded, then the decision processor 308 generates a blanking signal, indicating that a patient attachable electrode signal is at least potentially degraded, which conditions the blanking circuit 310 to blank the x lead signal. If the output signal from the comparator 306 indicates that the differential threshold was not exceeded, then the decision processor 308 generates a blanking signal which conditions the blanking circuit 310 to pass the ECG lead signal x unblanked. As used herein, a decision processor receives signals representing the results of comparing the differential patient attachable electrode signals to the differential threshold signal DT, and possibly representing other comparisons, and processes those signals to decide whether the ECG lead signal represented by that differential signal is potentially degraded and should be blanked.

More specifically, in the illustrated embodiment, for the ECG lead signal I, the $E_i$ input terminal is coupled to the $E_{LA}$ patient attachable electrode terminal and the $E_j$ input terminal is coupled to the $E_{RA}$ patient attachable electrode terminal. The blanking processor 206 generates a low frequency differential signal $DIFF_I(DC)$ corresponding to the ECG lead signal I, and representing the difference between the left arm patient attachable electrode signal $E_{LA}$ and the right arm patient attachable electrode signal $E_{RA}$. This low frequency differential signal $DIFF_I(DC)$ is compared to the differential threshold signal DT. If the differential threshold signal is exceeded, the signal indicating that the ECG lead signal I is potentially degraded is generated.

Similarly, for the ECG lead signal II, the $E_i$ input terminal is coupled to the $E_{LL}$ patient attachable electrode terminal and the $E_j$ input terminal is coupled to the $E_{RA}$ patient attachable electrode terminal. The blanking processor 206 generates a low frequency differential signal $DIFF_{II}(DC)$ corresponding to the ECG lead signal II, and representing the difference between the left leg patient attachable electrode signal $E_{LL}$ and the right arm patient attachable electrode signal $E_{RA}$. This low frequency differential signal $DIFF_{II}(DC)$ is compared to the differential threshold signal DT. If the differential threshold signal is exceeded, the signal indicating that the ECG lead signal II is potentially degraded is generated.

For the ECG lead signal V, the $E_i$ input terminal is coupled to the $E_{C1}$ patient attachable electrode terminal and the $E_j$ input terminal is coupled to receive the W signal. The blanking processor 206 also generates a low frequency differential signal $DIFF_V(DC)$ corresponding to the ECG lead signal V, and representing the difference between the first chest patient attachable electrode signal $E_{C1}$ and the W signal (i.e. the averaged signal derived from the left leg patient attachable electrode signal $E_{LL}$, the right arm patient attachable electrode signal $E_{RA}$ and the left arm patient attachable electrode signal $E_{LA}$). This low frequency differential signal $DIFF_V(DC)$ is compared to the differential threshold signal DT. If the differential threshold signal is exceeded, the signal indicating that the ECG lead signal V is potentially degraded is generated.

Similarly, for the ECG lead signal V+, the $E_i$ input terminal is coupled to the $E_{C2}$ patient attachable electrode terminal and the $E_j$ input terminal is coupled to receive the W signal. The blanking processor 206 generates a low frequency differential signal $DIFF_{V+}(DC)$ corresponding to the ECG lead signal V+, and representing the difference between the second chest patient attachable electrode signal $E_{C2}$ and the W signal (i.e. the averaged signal derived from the left leg patient attachable electrode signal $E_{LL}$, the right arm patient attachable electrode signal $E_{RA}$ and the left arm patient attachable electrode signal $E_{LA}$). This low frequency differential signal $DIFF_{V+}(DC)$ is compared to the differential threshold signal DT. If the differential threshold signal is exceeded, the signal indicating that the ECG lead signal V+ is potentially degraded is generated.

Fig. 3b illustrates an alternative to the blanking processor 206 illustrated in Fig. 3a. Those elements in Fig. 3b which are the same as those illustrated in Fig. 2 and Fig. 3a are designated by the same reference numbers and are not described in detail below. In Fig. 3b, the output terminal of the first buffer amplifier and LPF 204(a) is further coupled to a signal input terminal of a second comparator 210(a). A comparison input terminal of the second comparator 210(a) is coupled to a source of a second, absolute, threshold signal AT. The output terminal of the second buffer amplifier and LPF 204(b) is further coupled to a signal input terminal of a third comparator 210(b). A comparison input terminal of the third comparator 210(b) is also coupled to the source of the absolute threshold signal AT. The respective output terminals of the first, second and third comparators 306, 210(a) and 210(b) are coupled to corresponding input terminals of a decision processor 312. An output terminal of the decision processor 312 is coupled to the control input terminal of the blanking circuit 310.

In operation, the blanking processor 206 illustrated in Fig. 3b operates similarly to that illustrated in Fig. 3a. The second and third comparators 210(a) and 210(b) operate as described above with respect to Fig. 2. The respective low frequency or DC signals $E_i(DC)$ and $E_j(DC)$ from buffer amplifier and LPFs 204(a) and 204(b) are compared to the absolute threshold AT in the second and third comparators 210(a) and 210(b). As described above, if electrical contact between a patient attachable electrode RL, LL, RA, LA, C1, C2 and the patient is degraded, the common branch patient attachable electrodes are pulled toward ground. The absolute threshold AT is set to detect this condition. The decision processor 312 receives the results of the comparisons from the first, second and third comparators, 306, 210(a) and 210(b) respectively, and generates a blanking signal conditioning the blanking circuit 310 to blank the ECG x lead signal if the comparisons indicate that a corresponding threshold was exceeded. Otherwise, the decision processor 312 generates a blanking signal conditioning the blanking circuit to pass the ECG x lead signal unchanged.

More specifically, in the illustrated embodiment, for the ECG lead I signal, the left arm patient attachable electrode signal $E_{LA}$ is coupled to the first terminal $E_i$ and the right arm patient attachable electrode signal $E_{RA}$ is coupled to the second terminal $E_j$. The decision processor 312 generates a signal indicating that the ECG lead I signal is at least potentially degraded in response to at least one of: (a) the left arm patient attachable electrode signal $E_{LA}$ exceeding the predetermined absolute threshold AT signal; (b) the right arm patient attachable electrode signal $E_{RA}$ exceeding the predetermined absolute threshold AT signal; and (c) the differential signal $Diff_I(DC)$ exceeding the predetermined differential threshold DT signal (as described above with respect to Fig. 3a). Similarly, for the ECG lead II signal, the left leg patient attachable electrode signal $E_{LL}$ is coupled to the first terminal $E_i$ and the right arm patient attachable electrode signal $E_{RA}$ is coupled to the second terminal $E_j$. The decision processor 312 generates a signal indicating that the ECG lead II signal is at least potentially degraded in response to at least one of: (a) the left leg patient attachable electrode signal $E_{LL}$ exceeding the predetermined absolute threshold AT signal; (b) the right arm patient attachable electrode signal $E_{RA}$ exceeding the predetermined absolute threshold AT signal; and (c) the differential signal $Diff_{II}(DC)$ exceeding the predetermined differential threshold DT signal.

As described above, the ECG V and V+ chest lead signals are generated in response to the W signal, representing an averaged signal derived from Left Leg, Right Arm and Left Arm electrode signals $E_{LL}$, $E_{RA}$, $E_{LA}$. For the ECG lead V signal, the first chest patient attachable electrode signal $E_{C1}$ is coupled to the first terminal $E_i$ and the W signal is coupled to the second terminal $E_j$. The decision processor 312 generates a signal indicating that the ECG lead V signal is at least potentially degraded in response to at least one of: (a) the first chest patient attachable electrode signal $E_{C1}$ exceeding the predetermined absolute threshold signal; (b) the averaged W signal exceeding the predetermined absolute threshold signal; and (c) the differential signal $Diff_V(DC)$ exceeding the predetermined differential threshold signal. For the ECG lead V+ signal, the second chest patient attachable electrode signal $E_{C2}$ is coupled to the first terminal $E_i$ and the W signal is coupled to the second terminal $E_j$. The decision processor 312 generates a signal indicating that the ECG lead V+ signal is at least potentially degraded in response to at least one of: (a) the second chest patient attachable electrode signal $E_{C2}$ exceeding the predetermined absolute threshold AT signal; (b) the averaged W signal exceeding the predetermined absolute threshold AT signal; and (c) the differential signal $Diff_{V+}(DC)$ exceeding the predetermined differential threshold DT signal.

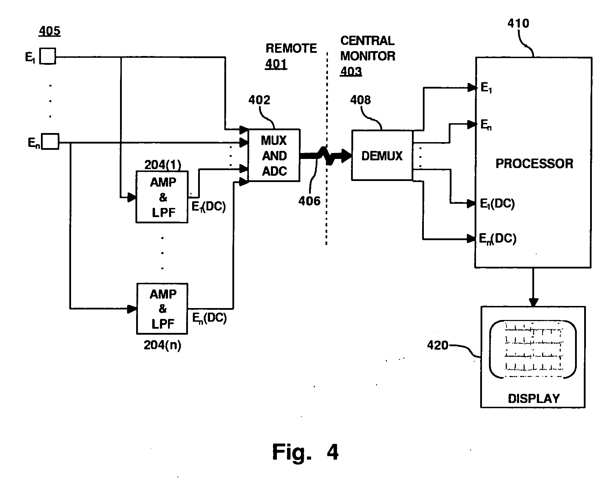

One skilled in the art understands that ECG monitoring systems often include a remote portion, for connecting patient attachable electrodes to a patient; and a central monitor, for receiving ECG signals from the remote portion, processing the received signals and displaying the resulting ECG lead signals on an image display device. Fig. 4 is a block diagram of a portion of a blanking processor 206 illustrated in Fig. 3a and Fig. 3b. In Fig. 4, those elements which are the same as those illustrated in Fig. 2 or Fig. 3 are designated by the same reference number and are not described in detail. In Fig. 4, a remote portion 401 is coupled to a central monitor 403 via a communications channel 406. In the illustrated embodiment, a plurality 405 of input terminals $E_1$ to $E_n$ are coupled to respective patient attachable electrode terminals, $E_{RL}$, $E_{LL}$, $E_{LA}$, $E_{RA}$, $E_{C1}$, $E_{C2}$. The plurality 405 of input terminals $E_1$ through $E_n$ are coupled to respective input terminals of a multiplexer (MUX) and analog-to-digital converter (ADC) 402 and to respective input terminals of respective buffer amplifiers and LPFs 204(1) through 204(n). Respective output terminals of the buffer amplifiers and LPFs 204(1) through 204(n) are coupled to corresponding further input terminals of the MUX and ADC 402. A digital output terminal of the MUX and ADC 402 is coupled through the communications channel 406 to a demultiplexer DEMUX 408 in the central monitor 403. Respective output terminals of the DEMUX 408 produce digital signals representing the multiplexed signals $E_1$ through $E_n$ and $E_1(DC)$ through $E_n(DC)$ and are coupled to corresponding input terminals of a processor 410. An output terminal of the processor 410 is coupled to an input terminal of a display device 420.

In operation, the MUX portion of 402 couples one of the plurality 405 of patient attachable electrode signals $E_1$ through $E_n$ or of the plurality of buffered and LPFed signals $E_1(DC)$ through $E_n(DC)$ input signal at a time to the ADC portion of 402. The ADC portion of 402 samples and converts that analog signal to a multibit digital electrical signal. These samples form a time division multiplexed multibit digital sample steam. This multibit digital sample stream is transmitted to the central monitor 403 via the communications channel 406.

The communications channel 406 may be implemented as a parallel digital data bus or a serial data connection. The digital samples may be accompanied by one or more clock signals to synchronize the operation of the central monitor 403 and the remote 401 or may be self clocking. The digital samples may also be transmitted in packetized form. The communications channel 406 may be a wired connection or a wireless connection. More specifically, in the illustrated embodiment, the communications channel may be an Ethernet or any other appropriate wired connection or a Wi-Fi or Bluetooth or any other appropriate wireless connection. The packets may be formatted according to TCP/IP formatting or any other appropriate packet protocol. One skilled in the art understands that a minimum sample rate must be maintained in order to provide the bandwidth necessary to process and display ECG lead signals derived from the patient attachable electrode signals $E_1$ through $E_n$. One skilled in the art also understands that the sample rate for the low frequency or DC patient attachable electrode signals $E_1(DC)$ through $E_n(DC)$ may be substantially lower than the sampling rate for the patient attachable electrode signals $E_1$ through $E_n$.

The DEMUX 408 extracts the digital data signals from the communications channel 406 and generates respective separate digital data streams for each patient attachable electrode signal $E_1$ through $E_n$ and for each low frequency or DC patient attachable electrode signal $E_1(DC)$ through $E_n(DC)$. These data streams are processed by the processor 410.

As used herein, a processor operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. The processor may operate with a display processor or generator. A display processor or generator is a known element for generating signals representing display images or portions thereof. A processor and a display processor comprises any combination of, hardware, firmware, and/or software.

7

An executable application as used herein comprises code or machine readable instructions for conditioning the processor to implement predetermined functions, such as those of an operating system, patient monitoring and/or treatment system or other information processing system, for example, in response user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

The processor 410 executes an executable procedure for receiving the respective digital data streams. The patient attachable electrode digital signals $E_1$ through $E_n$ are processed, as described above, to generate ECG lead signals. The low frequency or DC patient attachable electrode signals $E_1(DC)$ through $E_n(DC)$ are also processed as illustrated in Fig. 3 and described in more detail above to generate blanking signals for the ECG lead signals.

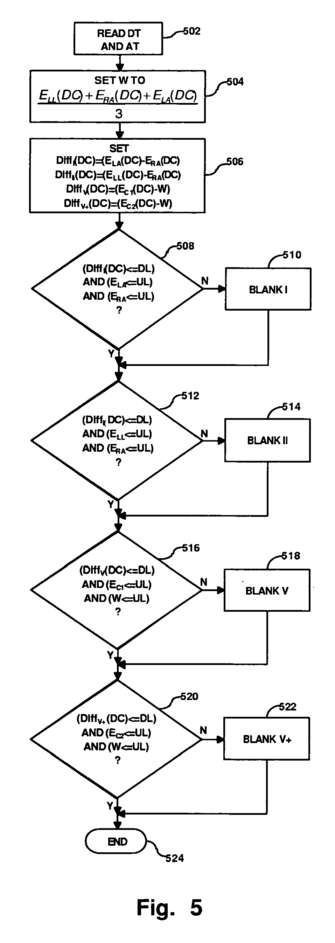

Fig. 5 is a flow chart illustrating the processing of patient attachable electrode signals performed by the processor 410 (Fig. 4) to determine connectivity of electrodes on a patient. In step 502 the differential threshold DT data and the absolute threshold AT data are read from storage. In step 504, the variable W is set to the average of the $E_{LL}(DC)$, $E_{RA}$(DC) and $E_{LA}(DC)$ signals. In step 506 respective differential signals $Diff_I(DC)$, $Diff_{II}(DC)$, $Diff_V(DC)$ and $Diff_{V+}(DC)$ are calculated for the ECG lead signals I, II, V and V+. The differential signal $Diff_I(DC)$ is compared to the differential threshold DT signal and the component patient attachable electrode signals $E_{LA}(DC)$ and $E_{RA}(DC)$ are compared to the absolute threshold signals AT. If the comparisons indicate that the signals are within the thresholds, the next ECG lead signal is checked in step 512. Otherwise the ECG lead signal I is blanked in step 510. In step 512, blanking for the ECG lead II signal is checked. The differential signal $Diff_{II}(DC)$ is compared to the differential threshold DT signal and the component patient attachable electrode signals $E_{LL}(DC)$ and $E_{RA}(DC)$ are compared to the absolute threshold signals AT. If the comparisons indicate that the signals are within the thresholds, the next ECG lead signal is checked in step 516. Otherwise the ECG lead signal II is blanked in step 514. In step 516, blanking for the ECG lead V signal is checked. The differential signal $Diff_V(DC)$ is compared to the differential threshold DT signal and the component patient attachable electrode signal $E_{C1}$ (DC) and the W signal are compared to the absolute threshold signals AT. If the comparisons indicate that the signals are within the thresholds, the next ECG lead signal is checked in step 520. Otherwise the ECG lead signal V is blanked in step 518. In step 520, blanking for the ECG lead V+ signal is checked. The differential signal $Diff_{V+}(DC)$ is compared to the differential threshold DT signal and the component patient attachable electrode signal $E_{C2}(DC)$ and the W signal are compared to the absolute threshold signals AT. If the comparisons indicate that the signals are within the thresholds, the executable procedure ends in step 524. Otherwise the ECG lead signal V+ is blanked in step 522.

One skilled in the art understands that in step 506, to simplify comparisons to thresholds, the absolute value of the differential signals $Diff_I(DC)$, $Diff_{II}(DC)$, $Diff_V(DC)$ and $Diff_{V+}(DC)$ may also be calculated. In steps 508, 512, 516 and 520, the absolute values of the differential signals $Diff_I(DC)$, $Diff_{II}(DC)$, $Diff_V(DC)$ and $Diff_{V+}(DC)$ may be compared to the appropriate thresholds.

The processor 410 then conditions a display generator to produce an image representative signal displaying the unblanked ECG lead signals and not displaying blanked ECG lead signals on the display device 420. One skilled in the art understands that additional displays may be generated and displayed for the user, such as visual indicators of which patient attachable electrode signals exceed the threshold and/or other parameters. Non-visual signals may also be generated such as audible alarms.

Setting of the absolute threshold AT and the differential threshold DT involves determining the constraints of the front end circuits, i.e. common mode voltage, DC differential voltage, rail voltages, etc., and analyzing the equivalent circuit of Fig. 2 to determine the patient attachable electrode resistances $R_{RL}$, $R_{RA}$, $R_{LA}$, $R_{LL}$, $R_{C1}$, $F_{C2}$, at which the constraints are violated. The absolute threshold AT signals and the differential threshold DT signals are set based on this analysis.

For example, in order to maintain the input signals of the differential amplifiers within the power rails, the DC patient attachable electrode signal must not become too low or too high in voltage. However, referring to Fig. 2, the equivalent circuit is very complex with six unknown resistances (the patient attachable electrode resistances $R_{RL}$, $R_{RA}$, $R_{LA}$, $R_{LL}$, $R_{C1}$, $R_{C2}$). A simple analytical solution to detecting an patient attachable electrode signal near a power rail is not available. In order to simplify the search for an absolute threshold AT signal which maintains operation within this constraint, three simplifications may be used. First, the patient attachable electrode resistances may be concurrently changed, i.e. increased equally, simulating a gradual drying of the patient attachable electrodes RL, LL, RA, LA, C1, C2. Second, the resistance of the reference, common branch, patient attachable electrode RL may be changed to simulate impaired electrical contact between the RL patient attachable electrode and the patient body. Third, the resistance of one of the non-common branch patient attachable electrodes LL, RA, LA, C1, C2 may be changed to simulate impaired electrical contact between the non-common branch electrode LL, RA, LA, C1, C2 and the patient body.

The equivalent circuit of Fig. 2 may be simulated to determine the respective patient attachable electrode signals $E_{RL}$, $E_{RA}$, $E_{LA}$, $E_{LL}$, $E_{C1}$, $E_{C2}$ resulting as changes are made to the patient attachable electrode resistances $R_{FL}$, $R_{RA}$, $R_{LA}$, $R_{LL}$, $R_{C1}$, $R_{C2}$, in the manner described above. Equations (1) through (5), below, are derived from Ohm's law and describe the DC operation of the circuit illustrated in Fig. 2. The patient attachable electrode resistances $R_{RL}$, $R_{RA}$, $R_{LA}$, $R_{LL}$, $R_{C1}$, $R_{C2}$ are initially set to zero ohms, and varied as described above. Equations (1) through (5) are solved to determine the resulting patient attachable electrode signals $E_{RL}$, $E_{RA}$, $E_{LA}$, $E_{LL}$, $E_{C1}$, $E_{C2}$. When a patient attachable electrode signal $E_{RL}$, $E_{RA}$, $E_{LA}$, $E_{LL}$, $E_{C1}$, $E_{C2}$ exceeds the power rail input constraint for the differential amplifier, the absolute threshold AT is set to a value to blank the ECG lead signals associated with that patient attachable electrode signal $E_{RL}$, $E_{RA}$, $E_{LA}$, $E_{LL}$, $E_{C1}$, $E_{C2}$ before that constraint is exceeded.

$$R_{COM} = R_{SC} + R_{RL} \text{ where } R_{SC} = 157.16K\Omega \tag{1}$$

$$R_{EQ} = R_{RA} + R_{BR} + R_{PD} \mid R_{LA} + R_{BR} + R_{PD} \mid R_{LL} + R_{BR} + R_{PD} \mid R_{C1} + R_{BR} + R_{PD} \mid R_{C2} + R_{BR} + R_{PD} \tag{2}$$

$$R_{EQ} = \cfrac{1}{\cfrac{1}{R_{RA} + R_{BR} + R_{PD}} + \cfrac{1}{R_{LA} + R_{BR} + R_{PD}} + \cfrac{1}{R_{LL} + R_{BR} + R_{PD}} + \cfrac{1}{R_{C1} + R_{BR} + R_{PD}} + \cfrac{1}{R_{C2} + R_{BR} + R_{PD}}} \tag{3}$$

$$E_X = REF \frac{R_{EQ}}{R_{COM} + R_{EQ}} \tag{4}$$

$$E_i = E_X \frac{R_{PD}}{R_{PD} + R_i} \text{ (for i=RA,LA,LL,C1,C2 )} \tag{5}$$

The maximum DC differential voltage input constraint on the differential amplifier may be simulated in a similar manner. In this case, as the patient attachable electrode resistances $R_{RL}$, $R_{RA}$, $R_{LA}$, $R_{LL}$, $R_{C1}$, $R_{C2}$ are changed in the manner described above, the circuit of Fig. 2 is simulated, using equations (1) through (5). Differential signals between patient attachable electrode signals $E_{RL}$, $E_{RA}$, $E_{LA}$, $E_{LL}$, $E_{C1}$, $E_{C2}$ appropriate for calculating the ECG lead signals (i.e. $E_{LA}(DC)-E_{RA}(DC)$, $E_{LL}(DC)-E_{RA}(DC)$, $E_{C1}(DC)-W$, $E_{C2}(DC)-W$) are calculated. When a differential signal exceeds the maximum DC differential voltage input constraint for the differential amplifier, the differential threshold DT is set to a value to blank the ECG lead signals associated with that differential signal before that constraint is exceeded.

It is further possible for the absolute threshold AT and differential threshold DT signals to be adaptively adjusted. For example, referring again to Fig. 4, the processor 410 makes the comparisons set out in Fig. 5. The processor 410 may further adjust the absolute threshold AT signals based on the results of the comparisons of the differential patient attachable electrode signals to the differential threshold DT signal. In this manner, the processor 410 may adaptively adjust the absolute threshold AT signal so that it does not result in unnecessarily blanking an ECG lead signal.

The present invention has been described by reference to an ECG patient monitoring system using six patient attachable electrodes to generate six ECG lead signals. However, one skilled in the art understands that the number of patient attachable electrodes and ECG leads is not germane. That is, an ECG patient monitor displaying 12 ECG lead signals using 10 patient attachable electrodes may also use the invention described above. Further, an ECG patient monitor displaying a subset of the ECG lead signals using a subset of patient attachable electrodes, such as a three ECG lead signal system, or an ECG patient monitor using a superset of ECG lead signals using additional patient attachable electrodes to provide additional views of the patient cardiac function, may also use the invention described above.

The steps illustrated in Fig. 5 and the functions provided by the circuitry illustrated in Fig. 3 and Fig. 4 may be performed by hardware specially designed and implemented to provide those functions, or by software, firmware software, or any combination of these.

Table 1 describes the variables and constants used above in describing the present invention.

**Table 1**

**Variables / Constants**

| | |
|---|---|
| $R_{COM}$ | Total Resistance in the common branch |
| $R_{EQ}$ | Equivalent resistance of the non-common branches |
| REF | Voltage driving the common lead branch |
| $E_X$ | Voltage appearing at divide point between common and non-common branches |
| $R_{PD}$ | Pull down resistance in non-common branches |
| $R_{RA}$ | Right Arm patient attachable electrode resistance |
| $R_{LA}$ | Left Arm patient attachable electrode resistance |
| $R_{LL}$ | Left Leg patient attachable electrode resistance |
| $R_{C1}$ | First Chest patient attachable electrode resistance |
| $R_{C2}$ | Second Chest patient attachable electrode res istance |
| $R_{BR}$ | Series non-common branch resistance. |
| $R_{SC}$ | Series common branch resistance. |
| DT | Differential Threshold |
| AT | Absolute Threshold |
| $E_{RA}$ | Right Arm patient attachable electrode voltage |
| $E_{LA}$ | Left Arm patient attachable electrode voltage |
| $E_{LL}$ | Left Leg patient attachable electrode voltage |
| $E_{C1}$ | First Chest patient attachable electrode voltage |
| $E_{C2}$ | Second Chest patient attachable electrode voltage |

**Claims**

1. An electrode connectivity determination system, coupleable to a plurality of patient attachable electrodes, comprising a first electrode (305) and a second electrode (315), **characterized by**:

   a first unity gain buffer amplifier for providing a first electrical signal from the first electrode (305);
   a second unity gain buffer amplifier for providing a second electrical signal from the second electrode (315);
   a first low pass filter (204a) for low pass filtering said first electrical signal and generating a first low frequency signal;
   a second low pass filter (204b) for low pass filtering said second electrical signal and generating a second low frequency signal;
   a subtractor (304) for generating a low frequency differential signal derived from said first low frequency signal and said second low frequency signal;
   a first comparator (306) for performing a first comparison of said low frequency differential signal with a first predetermined threshold signal; and
   a decision processor (308; 312) for generating a signal indicating an electrode signal is at least potentially degraded in response to said first comparison.

2. The system of claim 1 further comprising:

   a second comparator (210a; 210b) for performing a second comparison of one of said first and second low frequency signals with a second predetermined threshold signal; wherein:

   said decision processor (312) generates a signal indicating an electrode signal is at least potentially degraded in response to said first and said second comparisons.

3. The system of claim 2 wherein said low frequency differential signal and said first and second low frequency signals are respective substantially DC signals.

4. The system of claim 2 wherein said low frequency differential signal is derived by analog to digital conversion of said buffered and low pass filtered electrical signals.

5. The system of claim 2 comprised in an ECG monitor wherein said decision processor (308; 312) generates a signal indicating an electrode signal corresponding to ECG lead signal I is at least potentially degraded in response to:

said low frequency differential signal, being derived from data representing a difference between Left Arm and Right Arm patient attachable electrode signals, exceeding said first predetermined threshold signal; and
at least one of, (a) a Left Arm patient attachable electrode signal exceeding said second predetermined threshold signal, and (b) a Right Arm patient attachable electrode signal exceeding said second predetermined threshold signal.

6. The system of claim 2 comprised in an ECG monitor wherein said decision processor (308; 312) generates a signal indicating an electrode signal corresponding to ECG lead signal II is at least potentially degraded in response to:

said low frequency differential signal, being derived from data representing a difference between Left Leg and Right Arm patient attachable electrode signals, exceeding said first predetermined threshold signal; and
at least one of, (a) a Left Leg patient attachable electrode signal exceeding said second predetermined threshold signal, and (b) a Right Arm patient attachable electrode signal exceeding said second predetermined threshold signal.

7. The system of claim 2 comprised in an ECG monitor wherein said decision processor (308; 312) generates a signal indicating an electrode signal corresponding to a first ECG chest lead signal V is at least potentially degraded in response to:

said low frequency differential signal, being derived from data representing a difference between a First Chest patient attachable electrode signal and an averaged signal derived from Left Leg, Right Arm and Left Arm patient attachable electrode signals, exceeding said first predetermined threshold signal; and
at least one of, (a) the First Chest patient attachable electrode signal exceeding said second predetermined threshold signal and (b) said averaged signal exceeding said second predetermined threshold signal.

8. The system of claim 2 comprised in an ECG monitor wherein said decision processor (308; 312) generates a signal indicating an electrode signal corresponding to a second ECG chest lead signal V+ is at least potentially degraded in response to:

said low frequency differential signal, being derived from data representing a difference between said Second Chest patient attachable electrode signal and an averaged signal, derived from Left Leg, Right Arm and Left Arm patient attachable electrode signals, exceeding said first predetermined threshold signal; and
at least one of, (a) the Second Chest patient attachable electrode signal exceeding said second predetermined threshold signal and (b) said averaged signal exceeding said second predetermined threshold signal.

9. The system of claim 1 wherein a patient attachable electrode signal is at least potentially degraded if said patient attachable electrode signal is affected by impaired electrical contact between the patient attachable electrode (305; 315) and a patient (102) body.

10. The system of claim 9 wherein said impaired electrical contact occurs if at least one of, (a) said patient attachable electrode (305; 315) is detached from said patient (102) body and (b) electrical resistivity between said patient (102) attachable electrode (305; 315) and said patient body is significantly increased because of degraded electrical contact.

11. The system of claim 1 wherein said patient attachable electrodes (305; 315) are of a conventional 12 lead ECG signal set.

12. The system of claim 1 wherein said patient attachable electrodes (305; 315) are of a subset or reduced number of leads of a conventional 12 lead ECG signal set.

13. The system of claim 2 wherein said decision processor (308; 312) automatically adaptively adjusts said second predetermined threshold signal in response to said first comparison.

14. The system of claim 1 further comprising
a second comparator (210a; 210b) for performing a second comparison of one of said first and second low frequency signals with a second predetermined threshold signal; and
said decision processor (312) automatically adaptively adjusts said second predetermined threshold signal in response to said first comparison.

**15.** A method for determining connectivity status of a plurality of patient attachable electrodes, comprising:

generating a first low frequency signal by low pass filtering a first electrical signal from a first electrode of said patient attachable electrodes;
generating a second low frequency signal by low pass filtering a second electrical signal from a second electrode of said patient attachable electrodes;
generating a low frequency differential signal derived from at least said first low frequency signal and said second low frequency signal;
performing a first comparison of said low frequency differential signal derived from at least two patient attachable electrodes with a first predetermined threshold signal;
performing a second comparison of one of said first and second low frequency signals with a second predetermined threshold signal; and
generating a signal indicating an electrode signal is at least potentially degraded in response to said first and said second comparisons.

**Patentansprüche**

**1.** Elektroden-Verbindungszustands-Feststellungssystem, das mit einer Mehrzahl an einem Patienten anbringbarer Elektroden verbindbar ist, umfassend eine erste Elektrode (305) und eine zweite Elektrode (315), **gekennzeichnet durch**:

einen ersten Einheitsverstärkungs-Pufferverstärker zum Liefern eines ersten elektrischen Signals von der ersten Elektrode (305);
einen zweiten Einheitsverstärkungs-Pufferverstärker zum Liefern eines zweiten elektrischen Signals von der zweiten Elektrode (315);
ein erstes Tiefpassfilter (204a) zum Tiefpassfiltern des ersten elektrischen Signals und zum Erzeugen eine ersten Niederfrequenzsignals;
ein zweites Tiefpassfilter (204b) zum Tiefpassfiltern des zweiten elektrischen Signals und zum Erzeugen eines zweiten Niederfrequenzsignals;
einen Subtrahierer (304) zum Erzeugen eines Niederfrequenz-Differenzsignals, das aus dem ersten Niederfrequenzsignal und dem zweiten Niederfrequenzsignal erhalten wird;
einen ersten Komparator (306) zum Durchführen eines ersten Vergleichs des Niederfrequenz-Differenzsignals mit einem ersten vorbestimmten Schwellenwertsignal; und
einen Entscheidungsprozessor (308; 312) zum Erzeugen eines Signals, das anzeigt, dass ein Elektrodensignal mindestens potentiell ein schlechtes Signal ist, in Reaktion auf den ersten Vergleich.

**2.** System gemäß Anspruch 1, ferner umfassend:

einen zweiten Komparator (210a; 210b) zum Durchführen eines zweiten Vergleichs entweder des ersten oder des zweiten Niederfrequenzsignals mit einem zweiten vorbestimmten Schwellenwertsignal; wobei:

der Entscheidungsprozessor (312) in Reaktion auf den ersten und den zweiten Vergleich ein Signal erzeugt, das anzeigt, dass ein Elektrodensignal mindestens potentiell ein schlechtes Signal ist.

**3.** System gemäß Anspruch 2, wobei das Niederfrequenz-Differenzsignal und das erste und das zweite Niederfrequenzsignal jeweils im Wesentlichen Gleichstromsignale sind.

**4.** System gemäß Anspruch 2, wobei das Niederfrequenz-Differenzsignal durch Analog-Digital-Umwandlung der gepufferten und tiefpassgefilterten elektrischen Signale erhalten wird.

**5.** System gemäß Anspruch 2, integriert in einen EKG-Monitor, wobei der Entscheidungsprozessor (308; 312) ein Signal erzeugt, das anzeigt, dass ein Elektrodensignal, das einem EKG-Ableitungssignal I entspricht, mindestens potentiell ein schlechtes Signal ist, und zwar in Reaktion darauf:

dass das Niederfrequenz-Differenzsignal, das aus Daten erhalten wird, die eine Differenz zwischen Elektrodensignalen von einer an einem linken Arm und einer an einem rechten Arm eines Patienten anbringbaren Elektrode repräsentieren, das erste vorbestimmte Schwellenwertsignal übersteigt; und

dass mindestens entweder a) ein Elektrodensignal von einer an einem linken Arm eines Patienten anbringbaren Elektrode das zweite vorbestimmte Schwellenwertsignal übersteigt oder b) ein Elektrodensignal von einer an einem rechten Arm eines Patienten anbringbaren Elektrode das zweite vorbestimmte Schwellenwertsignal übersteigt.

6. System gemäß Anspruch 2, integriert in einen EKG-Monitor, wobei der Entscheidungsprozessor (308; 312) ein Signal erzeugt, das anzeigt, dass ein Elektrodensignal, das einem EKG-Ableitungssignal II entspricht, mindestens potentiell ein schlechtes Signal ist, und zwar in Reaktion darauf:

dass das NiederFrequenz-Differenzsignal, das aus Daten erhalten wird, die eine Differenz zwischen Elektrodensignalen von einer an einem linken Bein und einer an einem rechten Arm eines Patienten anbringbaren Elektrode repräsentieren, das erste vorbestimmte Schwellenwertsignal übersteigt; und

dass mindestens entweder a) ein Elektrodensignal von einer an einem linken Bein eines Patienten anbringbaren Elektrode das zweite vorbestimmte Schwellenwertsignal übersteigt oder b) ein Elektrodensignal von einer an einem rechten Arm eines Patienten anbringbaren Elektrode das zweite vorbestimmte Schwellenwertsignal übersteigt.

7. System gemäß Anspruch 2, integriert in einen EKG-Monitor, wobei der Entscheidungsprozessor (308; 312) ein Signal erzeugt, das anzeigt, dass ein Elektrodensignal, das einem ersten EKG-Brustwand-Ableitungssignal V entspricht, mindestens potentiell ein schlechtes Signal ist, und zwar in Reaktion darauf:

dass das Niederfrequenz-Differenzsignal, das aus Daten erhalten wird, die eine Differenz zwischen Elektrodensignalen von einer ersten an der Brustwand eines Patienten anbringbaren Elektrode und einem Durchschnittssignal aus Elektrodensignalen von an einem linken Bein, einem rechten Arm und einem linken Arm eines Patienten anbringbaren Elektroden repräsentieren, das erste vorbestimmte Schwellenwertsignal übersteigt; und

dass mindestens entweder a) ein Elektrodensignal von einer ersten an der Brustwand eines Patienten anbringbaren Elektrode das zweite vorbestimmte Schwellenwertsignal übersteigt oder b) das Durchschnittssignal das zweite vorbestimmte Schwellenwertsignal übersteigt.

8. System gemäß Anspruch 2, integriert in einen EKG-Monitor, wobei der Entscheidungsprozessor (308; 312) ein Signal erzeugt, das anzeigt, dass ein Elektrodensignal, das einem zweiten EKG-Brustwand-Ableitungssignal V+ entspricht, mindestens potentiell ein schlechtes Signal ist, und zwar in Reaktion darauf:

dass das Niederfrequenz-Differenzsignal, das aus Daten erhalten wird, die eine Differenz zwischen dem Elektrodensignal von einer zweiten an der Brustwand eines Patienten anbringbaren Elektrode und einem Durchschnittssignal aus Elektrodensignalen von an einem linken Bein, einem rechten Arm und einem linken Arm eines Patienten anbringbaren Elektroden repräsentieren, das erste vorbestimmte Schwellenwertsignal übersteigt; und

dass mindestens entweder a) das Elektrodensignal von einer zweiten an der Brustwand eines Patienten anbringbaren Elektrode das zweite vorbestimmte Schwellenwertsignal übersteigt oder b) das Durchschnittssignal das zweite vorbestimmte Schwellenwertsignal übersteigt.

9. System gemäß Anspruch 1, wobei ein Elektrodensignal von einer an einem Patienten anbringbaren Elektrode mindestens teilweise ein schlechtes Signal ist, wenn das Elektrodensignal von einer an einem Patienten anbringbaren Elektrode durch einen gestörten elektrischen Kontakt zwischen der an einem Patienten anbringbaren Elektrode (305; 315) und einem Körper eines Patienten (102) beeinträchtig wird.

10. System gemäß Anspruch 9, wobei der gestörte elektrische Kontakt auftritt, wenn mindestens entweder a) die an einem Patienten anbringbare Elektrode (305; 315) von dem Körper des Patienten (102) gelöst ist oder b) ein elektrischer Widerstand zwischen der an einem Patienten (102) anbringbaren Elektrode (305; 315) und dem Patientenkörper aufgrund eines schlechten elektrischen Kontakts beträchtlich erhöht ist.

11. System gemäß Anspruch 1, wobei die an einem Patienten anbringbaren Elektroden (305; 315) aus einem herkömmlichen 12-Kanal-EKG-Satz sind.

**12.** System gemäß Anspruch 1, wobei die an einem Patienten anbringbaren Elektroden (305; 315) aus einer Teilmenge oder aus einer verringerten Anzahl von Ableitungen eines herkömmlichen 12-Kanal-EKG-Satzes sind.

**13.** System gemäß Anspruch 2, wobei der Entscheidungsprozessor (308; 312) in Reaktion auf den ersten Vergleich das zweite vorbestimmte Schwellenwertsignal automatisch adaptiv einstellt.

**14.** System gemäß Anspruch 1, ferner umfassend:

einen zweiten Komparator (210a; 210b) zum Durchführen eines zweiten Vergleichs entweder des ersten oder des zweiten Niederfrequenzsignals mit einem zweiten vorbestimmten Schwellenwertsignal;
wobei der Entscheidungsprozessor (312) in Reaktion auf den ersten Vergleich das zweite vorbestimmte Schwellenwertsignal automatisch adaptiv einstellt.

**15.** Verfahren zum Feststellen eines Verbindungsstatus einer Mehrzahl an einem Patienten anbringbarer Elektroden, umfassend:

Erzeugen eines ersten Niederfrequenzsignals durch Tiefpassfiltern eines ersten elektrischen Signals von einer ersten Elektrode der an einem Patienten anbringbaren Elektroden;
Erzeugen eines zweiten Niederfrequenzsignals durch Tiefpassfiltern eines zweiten elektrischen Signals von einer zweiten Elektrode der an einem Patienten anbringbaren Elektroden;
Erzeugen eines Niederfrequenz-Differenzsignals, das mindestens aus dem ersten Niederfrequenzsignal und dem zweiten Niederfrequenzsignal erhalten wird;
Durchführen eines ersten Vergleichs des aus mindestens zwei an einem Patienten anbringbaren Elektroden erhaltenen Niederfrequenz-Differenzsignals mit einem ersten vorbestimmten Schwellenwertsignal;
Durchführen eines zweiten Vergleichs entweder des ersten oder des zweiten Niederfrequenzsignals mit einem zweiten vorbestimmten Schwellenwertsignal; und
Erzeugen eines Signals, das anzeigt, dass ein Elektrodensignal mindestens potentiell ein schlechtes Signal ist, in Reaktion auf den ersten und den zweiten Vergleich.

## Revendications

**1.** Système de détermination de connectivité d'électrodes, susceptible d'être couplé à une pluralité d'électrodes à attacher à un patient, comprenant une première électrode (305) et une seconde électrode (315),
**caractérisé par**
un premier amplificateur-tampon à gain unitaire pour fournir un premier signal électrique depuis la première électrode (305) ;
un second amplificateur-tampon à gain unitaire pour fournir un second signal électrique depuis la seconde électrode (315) ;
un premier filtre passe-bas (204a) pour filtrer en passe-bas ledit premier signal électrique et générer un premier signal à basse fréquence ;
un second filtre passe-bas (204b) pour filtrer en passe-bas ledit second signal électrique et générer un second signal à basse fréquence ;
un soustracteur (304) pour générer un signal différentiel à basse fréquence dérivé dudit premier signal à basse fréquence et dudit second signal à basse fréquence ;
un premier comparateur (306) pour effectuer une première comparaison dudit signal différentiel à basse fréquence avec un premier signal seuil prédéterminé ; et
un processeur de décision (308 ; 312) pour générer un signal indiquant qu'un signal d'électrode est au moins potentiellement dégradé en réponse à ladite première comparaison.

**2.** Système selon la revendication 1, comprenant en outre :

un second comparateur (210a ; 210b) pour effectuer une seconde comparaison de l'un parmi, ledit premier et ledit second signal à basse fréquence avec un second signal seuil prédéterminé ; dans lequel :

ledit processeur de décision (312) génère un signal indiquant qu'un signal électrode est au moins potentiellement dégradé en réponse à ladite première et ladite seconde comparaison.

**3.** Système selon la revendication 2, dans lequel ledit signal différentiel à basse fréquence et ledit premier et ledit second signal à basse fréquence sont des signaux respectifs sensiblement en courant continu.

**4.** Système selon la revendication 2, dans lequel ledit signal différentiel à basse fréquence est dérivé par conversion analogique/numérique dudit signal électrique tamponné et dudit signal électrique filtré en passe-bas.

**5.** Système selon la revendication 2, compris dans un moniteur d'électrocardiogramme, dans lequel ledit processeur de décision (308 ; 312) génère un signal indiquant qu'un signal d'électrode correspondant à un signal maître d'électrocardiogramme 1 est au moins potentiellement dégradé en réponse à :

ledit signal différentiel à basse fréquence, dérivé depuis des données représentant une différence entre un signal d'électrode à attacher au bras gauche d'un patient et un signal d'électrode à attacher au bras droit d'un patient, excède ledit premier signal seuil prédéterminé ; et
au moins une condition parmi : (a) un signal d'électrode à attacher au bras gauche du patient excède ledit second signal seuil prédéterminé, et (b) un signal d'électrode à attacher au bras droit du patient excède ledit second signal seuil prédéterminé.

**6.** Système selon la revendication 2, compris dans un moniteur d'électrocardiogramme dans lequel ledit processeur de décision (308 ; 312) génère un signal indiquant qu'un signal d'électrode correspondant à un signal maître d'électrocardiogramme II est au moins potentiellement dégradé en réponse à :

ledit signal différentiel à basse fréquence, dérivé depuis des données représentant une différence entre un signal d'électrode à attacher à la jambe gauche et un signal d'électrode à attacher au bras droit du patient, excède ledit premier signal seuil prédéterminé ; et
au moins une condition parmi : (a) un signal d'électrode à attacher à la jambe gauche du patient excède ledit second signal seuil prédéterminé,
et (b) un signal d'électrode à attacher au bras droit du patient excède ledit second signal seuil prédéterminé.

**7.** Système selon la revendication 2, compris dans un moniteur d'électrocardiogramme dans lequel ledit processeur de décision (308 ; 312) génère un signal indiquant qu'un signal d'électrode correspondant à un premier signal maître thoracique d'électrocardiogramme V est au moins potentiellement dégradé en réponse à :

ledit signal différentiel à basse fréquence, dérivé depuis des données représentant une différence entre un premier signal d'électrode à attacher au thorax d'un patient, et un signal moyen dérivé depuis des signaux d'électrodes à attacher à la jambe gauche, au bras droit et au bras gauche du patient, excède ledit premier signal seuil prédéterminé ; et
au moins une condition parmi : (a) le premier signal d'électrode à attacher au thorax du patient excède ledit second signal seuil prédéterminé, et (b) ledit signal moyen excède ledit second signal seuil prédéterminé.

**8.** Système selon la revendication 2, compris dans un moniteur d'électrocardiogramme dans lequel ledit processeur de décision (308 ; 312) génère un signal indiquant qu'un signal d'électrode correspondant à un second signal maître thoracique d'électrocardiogramme V+ est au moins potentiellement dégradé en réponse à :

ledit signal différentiel à basse fréquence, dérivé depuis des données représentant une différence entre ledit second signal d'électrode à attacher au thorax d'un patient et un signal moyen, dérivé depuis les signaux d'électrodes à attacher à la jambe gauche, au bras droit et au bras gauche du patient, excède ledit premier signal seuil prédéterminé ; et
au moins une condition parmi : (a) le second signal d'électrode à attacher au thorax d'un patient excède ledit second signal seuil prédéterminé, et (b) ledit signal moyen excède ledit second signal seuil prédéterminé.

**9.** Système selon la revendication 1, dans lequel un signal d'électrode à attacher à un patient est au moins potentiellement dégradé si ledit signal d'électrode à attacher à un patient est affecté par un contact électrique défectueux entre l'électrode à attacher au patient (305 ; 315) et le corps d'un patient (102).

**10.** Système selon la revendication 9, dans lequel ledit contact électrique défectueux se produit dans l'une au moins des situations suivantes : (a) ladite électrode à attacher au patient (305 ; 315) est détachée depuis le corps dudit patient (102), et (b) la résistivité électrique entre ladite électrode (305 ; 315) à attacher au patient (102) et le corps dudit patient est augmentée de manière significative à cause d'un contact électrique dégradé.

**11.** Système selon la revendication 1, dans lequel lesdites électrodes à attacher au patient (305 ; 315) sont celles d'un groupe traditionnel fournissant 12 signaux maître d'électrocardiogramme.

**12.** Système selon la revendication 1, dans lequel lesdites électrodes à attacher au patient (305 ; 315) sont un sous-groupe ou un nombre réduit de celles d'un groupe traditionnel fournissant 12 signaux maître d'électrocardiogramme.

**13.** Système selon la revendication 2, dans lequel ledit processeur de décision (308 ; 312) ajuste automatiquement de manière adaptative ledit second signal seuil prédéterminé en réponse à ladite première comparaison.

**14.** Système selon la revendication 1, comprenant en outre un second comparateur (210a ; 210b) pour effectuer une seconde comparaison de l'un parmi ledit premier et ledit second signal à basse fréquence avec un second signal seuil prédéterminé ; et
ledit processeur de décision (312) ajuste automatiquement de manière adaptative ledit second signal seul prédéterminé en réponse à ladite première comparaison.

**15.** Procédé pour déterminer l'état de connectivité d'une pluralité d'électrodes à attacher un patient, comprenant les étapes suivantes :

on génère un premier signal à basse fréquence par filtration en passe-bas d'un premier signal électrique depuis une première électrode desdites électrodes à attacher au patient ;
on génère un second signal à basse fréquence par filtration en passe-bas d'un second signal électrique depuis une seconde électrode desdites électrodes à attacher au patient ;
on génère un signal différentiel à basse fréquence dérivé au moins depuis ledit premier signal à basse fréquence et ledit second signal à basse fréquence ;
on effectue une première comparaison dudit signal différentiel à basse fréquence dérivé depuis au moins deux électrodes à attacher au patient avec un premier signal seuil prédéterminé ;
on effectue une seconde comparaison d'un signal parmi ledit premier et
ledit second signal à basse fréquence avec un second signal seuil prédéterminé ; et
on génère un signal indiquant qu'un signal d'électrode est au moins potentiellement dégradé en réponse à ladite première et à ladite seconde comparaison.

Fig. 1

Fig. 2

Fig. 3

**Fig. 4**

**Fig. 5**

**EP 1 611 845 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4459993 A